# EUROPEAN PATENT APPLICATION

(11) **EP 4 778 458 A1**
(43) Date of publication of application: **22.07.2026**
(21) Application number: 25152790.9
(22) Date of filing: 20.01.2025
(51) Int. Cl.: A61B 5/145

(54) **SUPPORTING ORAL CAVITY FRAME, APPARATUS AND METHOD USING THE SAME**

(71) Applicant: entoothiast AG, 8005 Zürich (CH)
(72) Inventor: Maurmann, Linus, 8055 Zürich (CH)
(74) Representative: Prins Intellectual Property AG

(57) **Abstract**

A supporting oral cavity frame for carrying a device (9, 9') and for placement within an oral cavity of a user comprising a wire-like frame structure (1) of resiliently flexible material; wherein the frame structure (1) comprises a central section (20) with at least one attachment area (2, 2') for permanently or releasably attaching the device (9, 9') to the supporting frame (1) and several wire-like segments (3, 4, 5, 5', 6); wherein the several wire-like segments (3, 4, 5, 5') extend from the central section (20) and each of the several extending wire-like segments (3, 4, 5, 5') forms a distal section (31, 41, 51, 51'); wherein the wire-like frame structure (1) with the several extending segments (3, 4, 5, 5') is dimensioned and bent such that, in an installed state of the supporting frame (1) within the oral cavity, the distal sections (31, 41, 51, 51') of the several extending segments (3, 4, 5, 5') rest against different areas of tissue within the oral cavity to stabilize the supporting oral cavity frame with the at least one attachment area (2, 2') at a desired location within the oral cavity.

## Description

### Technical Field

The invention relates to a supporting oral cavity frame for carrying a device and for placement within the oral cavity of a user. The invention further relates to an apparatus comprising a supporting oral cavity frame and a device and a method of placing a device within the oral cavity of a user.

### Technical Background

Devices placed in the oral cavity of a user are used for various medical, dental, fitness, or technological purposes. Sensors e.g. may be used to detect biomarkers such as glucose, cortisol, lactate or others (salivary biomarker sensors, saliva composition sensors), to monitor chemical composition of saliva, pH or temperature, or to measure vital parameters such as heart rate or heart rate variability. Other devices, such as drug-delivery devices may also be placed in the oral cavity.

When placing devices in the oral cavity general safety requirements must be addressed, including but not limited to the possibility of swallowing or chocking on the device or parts of the device, interference of the device with the respiratory system, disintegration of the device under increased external forces, or the possibility of biting on the device.

Different attachment systems addressing at least some of these safety concerns have been developed in the past, including a dental band, a dental bracket, a dental clasp, a dental splint, or a dental retainer. The same attachment systems may be used for drug delivery devices held in the mouth as described in EP3065808.

WO2022128439 describes a dental sensor for an intraoral region. The dental sensor comprises a housing with a flat or approximately anatomically preformed tooth-facing contact surface covered with a sheet-like layer of a mouldable and plastic material for attaching the sensor directly to one or more tooth. The sensor device cannot be readily removed and is easily swallowed if it accidentally falls off.

EP1768503 describes a device for determining when a user engages in smoking. The device is placed in the oral cavity by fixing it to the user's teeth or bony structure in the oral cavity. For tooth-based fixation, the attachment point or points may be lingual or buccal, depending on the desired location of the device. For bony structure fixation, bone screw(s) may be placed in the hard palate, maxilla, mandible or other bony structure. Thus, the attachment is customized for each user and requires professional assistance.

EP3097411 describes a mouth-based salivary biosensor integrated into a mouthguard. Mouthguards are typically polymeric articles designed to fit firmly and snuggly over the teeth. US2017095204, EP3471599, EP3904850 and WO21055433 also disclose sensors for placement in the oral cavity via mouthguards, retainers, aligners or splints.

Although, such devices can be easily placed and removed without professional assistance, they need individual customization to the user's teeth.

EP3740155 and WO21155285 describe a sensor device attached to a tooth via a molar band. The band does not need customization but cannot be placed and removed by the user. Professional assistance is required. The sensor is easily swallowed if it accidentally falls off the tooth.

WO2021250664 describes an intraoral device attached to one or more tooth via a clip. The band requires customization and cannot be placed and removed by the user. Professional assistance is required. The device is easily swallowed if it accidentally falls off the tooth.

Thus, all the known solutions to place sensors or other devices in the oral cavity require (i) a specific customization of the device to the user's teeth or oral anatomy (e.g. retainer, splint) and/or (ii) professional assistance by a dentist or dental assistant for securely placing the device in the oral cavity (e.g. band, bracket or clasp).

Next to the general safety requirements, other factors play also a role for the design of the device and/or the attachment. The factors may be: avoidance of individual customization; ability for insertion and removal by the user and the time required to do so; flow of saliva should not be hindered; asymmetry of the dental structure should be taken into account; avoidance of volumetric change in the oral cavity to not interfere with air flow while breathing; avoidance of interference of the device with verbal expression, when swallowing saliva or when drinking; ensuring high level of comfort even when sleeping in a lateral position; avoidance of damage on the device or teeth in episodes of bruxism during sleep. Furthermore, attaching a sensor to the teeth for a prolonged time can lead to lasting change in tooth position or general jaw misalignment.

### Summary of the Invention

It is an objective of the invention to provide a novel means for securely placing a device, e.g. a sensor or monitoring device or a drug-delivery device, within an oral cavity of a user, wherein the novel means do not require individual customization and/or the assistance of professionals, such as dentist or dental assistant, for the placement of the device. It is a further objective that the above-described safety requirements and at least some of the further factors to ensure comfortable placement of a sensor or a drug delivery device are fulfilled.

At least one of the objectives of the present invention is achieved by a supporting oral cavity frame for carrying a device and for placement within an oral cavity of a user according to claim 1, an apparatus according to claim 12 or a method according claim 15.

The supporting oral cavity frame for carrying a device and for placement within an oral cavity of a user comprises a wire-like frame structure of resiliently flexible material. The frame structure comprises a central section with at least one attachment area for permanently or releasably attaching the device to the supporting frame and comprises several wire-like segments. The several wire-like segments extend from the central section and each of the several extending wire-like segments forms a distal section. The wire-like frame structure with the several extending segments is dimensioned and bent such that, in an installed state of the supporting frame within the oral cavity, the distal sections of the several extending segments rest against different areas of tissue, mainly soft tissue, within the oral cavity to stabilize the supporting oral cavity frame with the at least one attachment area at a desired location within the oral cavity.

The device may be a sensor or monitoring device or a drug-delivery device. The device may be an electrical or electronic device or biochemical device. A biochemical device may rely on biochemical reactions without the need of electronics.

Contrary to the known attachment means, which are directly attached to teeth or braced between or around teeth via a mouthguard or retainer, the present invention makes use of the volume and geometry of the oral cavity, which is less specific to the user. The three segments, which extend from a proximal or central section, form arms reaching distally (as seen from the proximal or central section) into different directions and regions of the oral cavity and rest against (soft) tissue, different from the teeth. The supporting oral cavity frame is held in place merely by the extending segments spreading in different directions and its distal sections resting against the tissue with a small force created by the resiliently flexible material. Thus, the supporting oral cavity frame has a spring-like structure and is designed to snuggly fit into an oral cavity of a user. Its distal sections span over typically 50 to 80 mm. The extending segments may be approximately 20 to 50 mm long, i.e. extend about that length from the attachment area or the device. All sorts of small devices, which may have a typical size (length, width) of 15 to 30 mm and thickness of 3 to 8 mm, may be attached to the central or proximal attachment area.

The attachment areas may be formed by or at connection points of two wire-like segments or junctions of more than two wire-like segments within the central section. The attachment areas may also be formed by bends (e.g. of approx. 90 degrees) or loops (e.g. of approx. 180 degrees) in the wire-like segments within the central section or by platforms formed by or attached to the wire-like segment. The device may be attached to these connection points, junctions, bends or loops in the wire-like segments or to the platforms fixed to the wire-like segments. Thus, the central section may be defined as the section where the device will be attached and where the wire-like segments are connected to each other. The wire-like frame structure may comprise two or more wires connected to each other through a case or housing of the device at an attachment area. Alternatively, the wires may also be connected through glueing, soldering or spot welding.

The supporting oral cavity frame, which is mainly formed by interconnected wire-like segments to form a wire-like frame structure of resiliently flexible material, is optimized to the volume and geometry of the oral cavity and may be produced in different sizes to suit individuals of different ethnicity, gender, size and age. No customization is necessary because the supporting oral cavity frame is not attached to the teeth of the user but is designed to fit to a few common sizes of the oral cavity. In addition, the supporting oral cavity frame can be easily inserted or removed by the user and professional assistance is not required. Thus, the supporting oral cavity frame is user-insertable and user-removable. In other words, the supporting oral cavity frame is self-exchangeable by the user. The user is typically a human being but can also be an animal or pet animal. In the case of an animal, the supporting oral cavity frame may be inserted or removed by a person. Furthermore, the supporting oral cavity frame is large enough that it cannot be swallowed by the user; it does not interfere with the respiratory system; and due to its flexibility, it does not interfere with verbal expression or when drinking because the mouth can be easily opened and closed while the supporting oral cavity frame is inserted. Due to its spring-like nature it adjusts to changes in geometry of the oral cavity when opening and closing the mouth.

Further embodiments of the invention are set forth in the dependent claims.

In some embodiments, at least three wire-like segments extend from the central section and each of the at least three extending wire-like segments forms a distal section. The wire-like frame structure with the at least three extending segments is dimensioned and bent such that, in an installed state of the supporting frame within the oral cavity, the distal sections of the at least three extending segments rest against three different areas of tissue within the oral cavity to stabilize the at least one attachment area at a desired location within the oral cavity. At least three extending segments further stabilize the positioning of the supporting oral cavity frame as compared to a version with only two extending segments, because the risk of tilting the entire frame is considerably reduced.

In some embodiments, the distal sections of the wire-like extending segments may be formed by free ends or loops. If a distal section is a free end of a wire-like extending segment it may be advantageous that the free end is provided with a protecting sleeve to minimize the risk of irritating the tissue when wearing the supporting oral cavity frame. The free end may also be bent backwards by 180 degrees to obtain a more rounded free end. On the other hand, the wire-like extending segment may be formed as a larger loop or turn enlarging the area of the distal section which rests against the tissue. The loop or turn may be in the range of 90 to 360 degrees. The frame structure may be coated with a biocompatible material, preferably a soft and elastic material safe for intra-oral use. The protecting sleeve may be of a biocompatible material safe for intra-oral use.

In some embodiments, the frame structure may comprise a fourth extending segment forming a distal section for resting against an area of tissue. The fourth distal section of the fourth extending segment may be near one of the at least three distal sections.

In some embodiments, the wire-like extending segments extend in different directions from the central section. Thus, the central section lies approximately in the centre of the frame structure and is supported by it in at least three different directions.

In some embodiments, the wire-like frame structure may comprise an intermediate wire-like segment connecting two attachment areas and/or extending wire-like segments.

In some embodiments, the at least one attachment area may be formed by or at connection points or junctions of the wire-like segments or by bends or loops in the wire-like segment within the central section. The attachment area may be formed by a platform formed by or attached to the wire-like segment. A housing of the device may be attached to the platform or directly to the wire-like segment of the frame structure of the supporting oral cavity frame. Junctions are formed where at least two wire-like segments connect. These junctions may form attachment areas for the device. Thus, the central section may be defined as the section where the device will be attached and where the wire-like segments are connected to each other.

In some embodiments, the wire-like segments may comprise U-turn loops to form pivot points increasing resilient flexibility within the frame structure. The U-turn loops or pivot points influence the kinetic behaviour of the supporting oral cavity frame and allow to create a virtual pivot point near the rotational centre of the temporomandibular joint to parallel the motion of a moving jaw. The U-turn loops may be formed by the intermediate wire-like segment or by an extending wire-like segment. The U-turn loops may be formed within a looped distal section of an extending wire-like segment.

In some embodiments, the frame structure may be made of elastically deformable material exhibiting sufficient tension force for holding the supporting oral cavity frame in place within the oral cavity. Such a frame structure may be bent when inserting it in the oral cavity and may also adjust the movements of the jaw while wearing the supporting oral cavity frame. The elastically deformable material may be spring steel, shape memory alloy, shape memory polymer, elastomer including thermoplastic elastomer, flexible fibre-reinforced composite. The material should be suitable for intra-oral applications.

In some embodiments, the frame structure may be made from one bent wire or several bent wires connected to each other, preferably a spring steel wire. The bent wires may have a diameter in the range of 0.3 to 1.2 mm.

In some embodiments, the frame structure may be 3D-printed.

In some embodiments, the frame structure may be coated with a biocompatible material, preferably a soft and elastic material.

In some embodiments, the wire-like frame structure with two extending wire-like segments is dimensioned and bent such that, in the installed state of the supporting frame within the oral cavity, a first distal section of a first extending segment may be accommodated in an upper anterior vestibule of the user and a second distal section of a second extending segment may be accommodated in a lower anterior vestibule of the user. When correctly inserted into to the oral cavity, the supporting oral cavity frame is thus stabilized by the at least two extending segments reaching to different regions in the oral cavity, i.e. near the front of the upper and lower jaw. Because the frame structure is of resiliently flexible material, the frame structure can adjust to movements of the jaw.

In some embodiments, the wire-like frame structure with at least three extending wire-like segments is dimensioned and bent such that, in the installed state of the supporting frame within the oral cavity, a first distal section of a first extending segment may be accommodated in an upper anterior vestibule of the user, a second distal section of a second extending segment may be accommodated in a lower anterior vestibule of the user, and a third distal section of a third extending segment may be accommodated in a posterior vestibule of the user. When correctly inserted into to the oral cavity, the supporting oral cavity frame is thus stabilized by the at least three extending segments reaching to different regions in the oral cavity, i.e. near the front of the upper and lower jaw and in the back in a region near the molars (e.g. on the skin-covered bone on the maxillary third molar). Because the frame structure is of resiliently flexible material, the frame structure can adjust to movements of the jaw.

In some embodiments, the central section lies approximately in a plane and the extending segments with the distal sections are bent in the same direction out of the plane. Thereby, the first distal section and the second distal section may slightly reach around the gingiva towards the canines or incisors. Thus, the supporting oral cavity frame is adjusted to the shape of the right or left vestibule of the oral cavity.

In some embodiments, the wire-like frame structure with the at least three extending wire-like segments is dimensioned and bent such that, in the installed state of the supporting frame within the oral cavity, a first distal section of a first extending segment is accommodated in an upper left anterior vestibule of the user, a second distal section of a second extending segment is accommodated in an upper right anterior vestibule of the user, and a third distal section of a third extending segment is accommodated sublingual or at the palate. The first and second distal section may be formed by free ends of the first and second extending segment, respectively. The third distal section may be formed by a loop or turn of the third extending segment connecting to the first and second extending segments. The attachment area may be arranged between the first and the third extending segments, between the second and the third extending segments or on both sides. When correctly inserted into to the oral cavity, the supporting oral cavity frame is thus stabilized by the at least three extending segments reaching to different regions in the oral cavity, i.e. near the right and left front of the upper jaw and with a loop in a sublingual region or at the hard palate. Because the frame structure is of resiliently flexible material, the frame structure can adjust to movements of the jaw.

The invention further refers to an apparatus comprising the supporting oral cavity frame as described above and a device attached to the at least one attachment area within the central section of the frame structure, wherein the device may be an electrical or electronic device or a biochemical device. The device may be a sensor or monitoring device or a drug delivery device.

In some embodiments of the apparatus, the device may comprise at least one housing, a sensor unit or a drug-delivery unit, a battery for operating the sensor unit, an induction coil for charging the battery, and a processing unit. The device may comprise one single housing attached to one attachment area, or two separate housings attached to two separate attachment areas and connected via an electrical wire. The housing(s) may be sealed and/or coated to protect the electronics. The variant with two housings allows to create a more resiliently flexible structure of the supporting oral cavity frame because the attachment areas may be connected via an intermediate wire-like segment with additional turns or loops.

The invention further refers to a method of placing a device, preferably an electrical or electronic device or a biochemical device, within an oral cavity of a user. The method comprises the steps of: (a.) providing a supporting oral cavity frame comprising a wire-like frame structure of resiliently flexible material with wire-like segments ; (b.) attaching the device to the wire-like frame structure to obtain an apparatus; and (c.) placing the supporting oral cavity frame within the oral cavity such that extending sections of the wire-like segments rest against different areas of (soft) tissue within the oral cavity to stabilize the device at a desired location within the oral cavity of the user. The device may be used for various medical, dental, fitness, or technological purposes, e.g. a sensor or monitoring device or a drug delivery device. Sensors e.g. may be used to detect biomarkers such as glucose, cortisol, lactate or others (salivary biomarker sensors, saliva composition sensors), to monitor chemical composition of saliva, pH or temperature, or to measure vital parameters such as heart rate or heart rate variability. The supporting oral cavity frame may be a supporting oral cavity frame as described above. The apparatus may be an apparatus as described above.

Contrary to the known method of placing a device, such as a sensor or monitoring device or a drug-delivery device, where a support is directly attached to teeth or braced between or around teeth via a mouthguard or retainer, the present invention makes use of the volume and geometry of the oral cavity, which is less specific to the user. The segments, which extend from a proximal or central section, form arms reaching distally into different directions and regions of the oral cavity and rest against (soft) tissue, different from the teeth. The supporting oral cavity frame is held in place by its spring-like structure, i.e. merely by the extending segments spreading in different directions and its distal sections resting against the tissue with a small force created by the resiliently flexible material.

In some embodiments of the method, the apparatus may be placed in the right or left vestibule of the oral cavity of the user in that a first distal section of a first extending segment may be accommodated in an upper anterior vestibule of the user, a second distal section of a second extending segment may be accommodated in a lower anterior vestibule of the user, and optionally a third distal section of a third extending segment may be accommodated in a posterior vestibule of the user. When correctly inserted into to the oral cavity, the supporting oral cavity frame is thus stabilized by the several extending segments reaching to different regions in the oral cavity, i.e. near the front of the upper and lower jaw and optionally in the back in a region near the molars (e.g. on the skin-covered bone on the maxillary third molar). Because the frame structure is of resiliently flexible material, the frame structure has a spring-like structure and can adjust to movements of the jaw.

In some embodiments of the method, the apparatus may be placed within the oral cavity in that a first distal section of a first extending segment is accommodated in an upper left anterior vestibule of the user, a second distal section of a second extending segment is accommodated in an upper right anterior vestibule of the user, and a third distal section of a third extending segment is accommodated sublingual or at the palate. The first and second distal section may be formed by free ends of the first and second extending segment, respectively. The third distal section may be formed by a loop or turn of the third extending segment connecting to the first and second extending segments. The attachment area may be arranged between the first and the third extending segments, between the second and the third extending segments or on both sides. When correctly inserted into to the oral cavity, the supporting oral cavity frame is thus stabilized by the at least three extending segments reaching to different regions in the oral cavity, i.e. near the right and left front of the upper jaw and with a loop in a sublingual region or at the palate. Because its spring-like nature, the frame structure of resiliently flexible material can adjust to movements of the jaw.

### Brief Explanation of the Figures

The invention is described in greater detail below with reference to embodiments that are illustrated in the figures. The figures show:
- Fig. 1 a: first variant of a supporting oral cavity frame for placement in the right oral vestibule, under (a) a front view, under (b) a side view and under (c) a top view with respect to the head of a user;
- Fig. 2 a: second variant of a supporting oral cavity frame for placement in the right oral vestibule, under (a) a front view, under (b) a side view and under (c) a top view with respect to the head of a user;
- Fig. 3 a: third variant of a supporting oral cavity frame for placement in the right oral vestibule, under (a) a front view, under (b) a side view and under (c) a top view with respect to the head of a user;
- Fig. 4: a fourth variant of a supporting oral cavity frame for placement in the right oral vestibule, under (a) a front view, under (b) a side view and under (c) a top view with respect to the head of a user;
- Fig. 5: a 3D-printed prototype of an apparatus for testing purposes with a fifth variant of a supporting oral cavity frame for placement in the right oral vestibule, under (a) a front view, under (b) a side view and under (c) a top view with respect to the head of a user;
- Fig. 6: a side view of the variant of Fig. 3 within the oral cavity of a user;
- Fig. 7: a variant of a supporting oral cavity frame for sublingual placement under (a) a front view, under (b) a side view and under (c) a top view;
- Fig. 8: an apparatus with the supporting oral cavity frame of Fig. 4;
- Fig. 9: different shapes and positions of the device in the attachment section of the frame structure of Fig. 1;
- Fig. 10: different shapes of the device in the attachment section of the frame structure of Fig. 7;
- Fig. 11: different shapes and positions of the device in the attachment section of the frame structure of Fig. 3;
- Fig. 12: different variants of U-turn loops to increase and adjust flexibility of the frame structure.

### Embodiments of the Invention

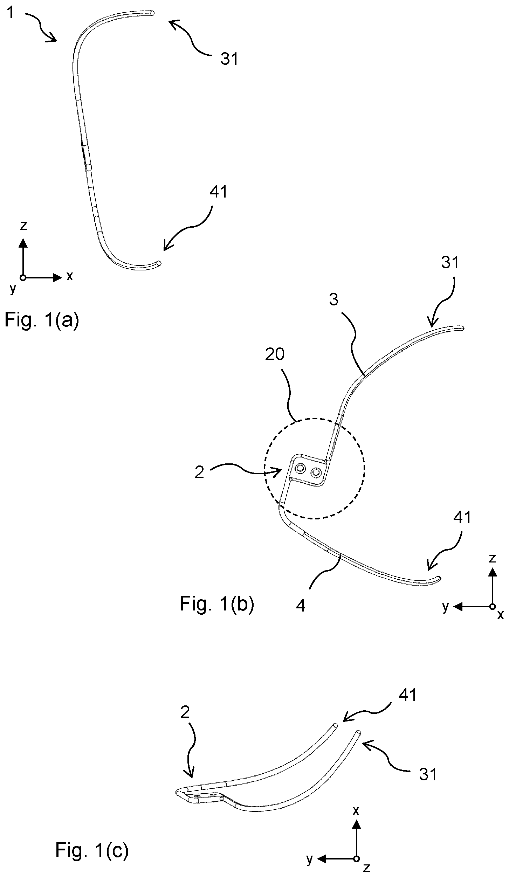

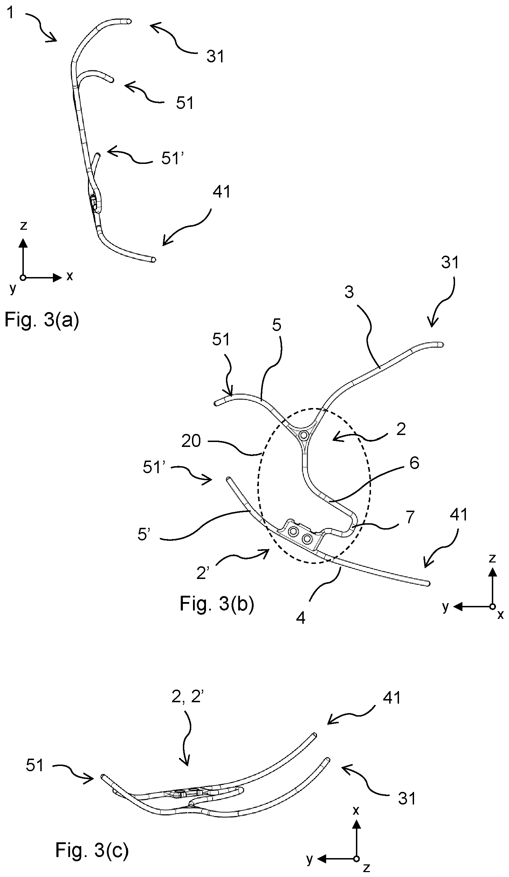

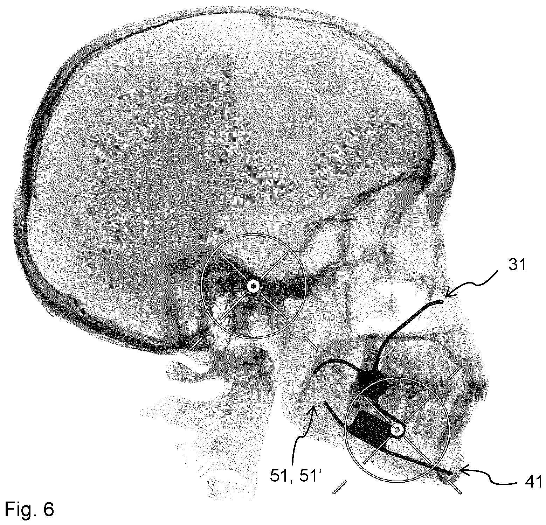

Figs. 1 to 5 show different variants of a supporting oral cavity frame for carrying a device (not shown) and for placement in a right oral vestibule of an oral cavity of a user. It is shown, under (a) a front view, under (b) a side view and under (c) a top view with respect to the head of a user. The views correspond to the installed situation when the supporting oral cavity frame is place in the right oral vestibule of a user. Thus, the front view is the view onto the face of the user. The side view is the view onto the right cheek of the user and the top view is the view on the top of the head. Fig. 6 shows a side view (i.e. onto the right cheek) of the variant of Fig. 3 in a user. Thus, directions are given with respect to the installed state within an oral cavity.

The supporting oral cavity frame of all four variants comprises a wire-like frame structure 1 of resiliently flexible material. An ideal material is spring steel wires, which can be readily bent and when bent hold their predefined structure but are still resiliently flexible.

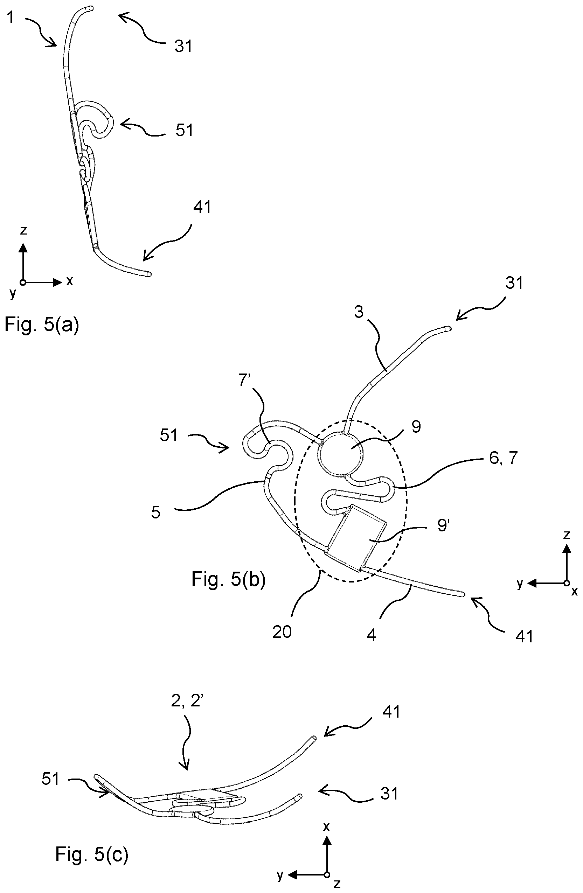

The frame structure 1 forms a central section 20 (dashed circle) with at least one attachment area 2, 2', to which a device (not shown) can be permanently or releasably attached. A variant with an attached device 9, 9' is shown in Fig. 5.

The attachment area can be a platform (as shown under reference sign 2) or can simply be the area, where parts of the wire-like structure connect. The attachment area can also be formed by a bend in the wire-like structure as e.g. the reference number 2' shows. In Fig. 1(b), the central or proximal attachment section 20 with the attachment area(s) is depicted by the dashed circle in the centre of the frame structure 1.

The frame structure 1 comprises several wire-like segments 3, 4, 5 extending in different directions from the central section 20. Free ends of these extending segments 3, 4, 5 form at least three distal sections 31, 41, 51 reaching into different regions of the oral cavity of the user and rest in these regions against the tissue. Thus, in the installed state, the supporting oral cavity frame is not firmly attached to teeth but is held in place merely by the extending wire-like segments.

The variant of Fig. 1, which is designed for the right vestibule of the oral cavity, has a first extending segment 3 reaching upwards to the front such that the first distal section 31 of the first extending segment 3 is accommodated in an upper anterior vestibule of the user. A second extending segment reaches downwards to the front such that the second distal section 41 of the second extending segment 4 is accommodated in a lower anterior vestibule of the user. The central section 20 with the attachment area 2 for the device is located in the vestibule near the molars.

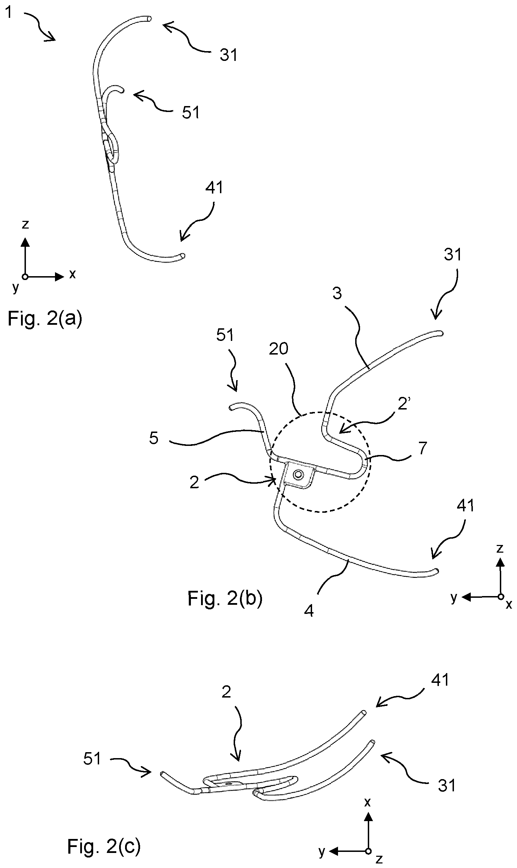

The variant of Fig. 2, which is designed for the right vestibule of the oral cavity, has a first extending segment 3 reaching upwards to the front such that the first distal section 31 of the first extending segment 3 is accommodated in an upper anterior vestibule of the user. A second extending segment reaches downwards to the front such that the second distal section 41 of the second extending segment 4 is accommodated in a lower anterior vestibule of the user. A third extending segment 5 reaches backwards such that the third distal section 51 of the third extending segment 5 is accommodated in a posterior vestibule of the user. The central section (20) (or the device when it is attached) is located approximately in the centre of the right vestibule.

The central section 20 with the at least one attachment area 2, 2' lies approximately in a plane as can be seen in Fig. 2(a) or Fig. 2(c) and which corresponds to the contour inside the cheek of the user. The distal sections 31, 41, 51 reach out of the plane and are bent inside towards the tongue. The extending segments 3, 4, 5 with the distal sections 31, 41, 51 are adapted to the contour of the oral cavity and slightly reach around the gingiva or and a region near the molars (e.g. on the skin-covered bone on the maxillary third molar) to form kind of a hook for stabilizing the position of the supporting oral cavity frame.

The first extending segment 3 also forms a U-turn loop 7 near or within the central section 20. Such a U-turn loop 7 increases the flexibility of the frame structure 1 allowing the first and second extending segments 3, 4 to adapt to the changing distances between the upper and lower anterior vestibule when the mouth is opened and closed.

The variant of the supporting oral cavity frame as shown in Fig. 3 differs from the variant of Fig. 2 in that it comprises a fourth extending segment 5' with a fourth distal section 51' in the form of a free end. The fourth extending segment 5' also extends from a central section 20 and reaches backwards such that the fourth distal section 51' of the fourth extending segment 5' is also accommodated in a posterior vestibule of the user like the third distal section 51.

The variant has a central section 20 with two attachment areas 2, 2' formed at a connection of the first and third extending segments 3, 5 and at a connection of the second and fourth extending segments 4, 5'. An intermediate wire-like segment 6 connects the two attachment areas 2, 2'.

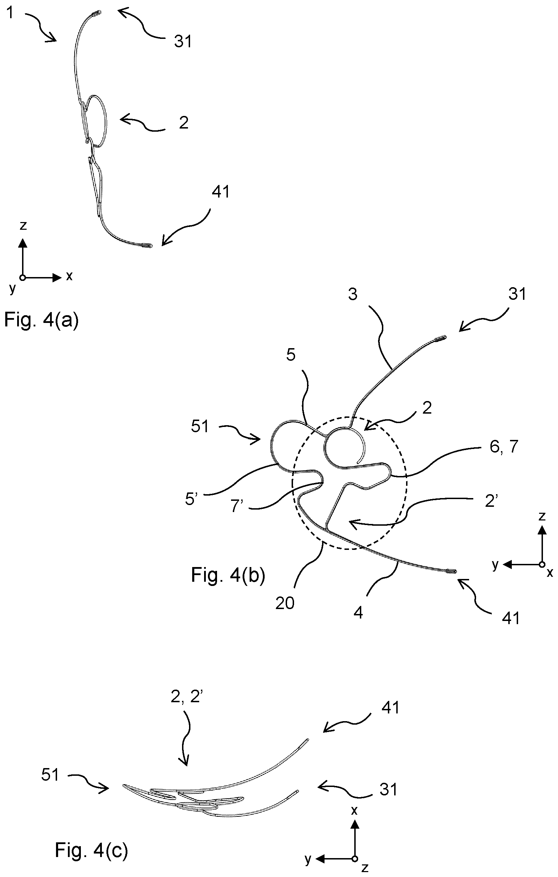

The variant of the supporting oral cavity frame as shown in Fig. 4 differs from the variant of Fig. 2 in that the distal section 51 of third extending segment 5 is formed as a loop such that the third extending segment 5 is connected to the attachment area 2, 2' or junctions within the central section 20 at both ends. The variant of Fig. 4 also differs from the variant of Fig. 3 in that the third extending segment 5 and the fourth extending segment 5' are connected to each other to form the distal section 51. The distal section 51 in the form of a loop has the advantage that fewer wires are necessary to manufacture the frame structure 1. It also optimizes the kinematic behaviour because pivot points can be adjusted to better reflect movement of the jaws. Furthermore, it covers a larger area for resting against the tissue. The variant as shown in Fig. 4 may be made of two bent spring steel wires. A first wire forming the first extending segment 3 and the intermediate segment 6 and a second wire forming the second and third extending segments 4, 5.

The frame structure 1 comprises a central section 20 with two attachment areas 2, 2' or junctions between extending segments 3, 4, 5, 5'. The frame structure 1 further comprises an intermediate segment 6 comprising a U-turn loop 7 to increase flexibility. The third extending segment 5 is also provided with a U-turn loop 7' to increase flexibility.

The free ends of the first and second extending segments 3, 4 are provided with protecting sleeves.

Fig. 5 shows schematic drawing of an apparatus comprising a supporting oral cavity frame similar to the one in Fig. 4 and a device including two housings 9, 9'. The device with the two housings are depicted as square boxes how it was 3D-printed for testing purposes. The variant of the supporting oral cavity frame as shown in Fig. 5 differs from the variant in Fig. 4 in that the third distal section 51 is formed by two consecutive U-turn loops 7'. While these U-turn loops 7' provide a section to rest against tissue in the posterior vestibule, they also increase flexibility of the frame structure 1. The intermediate segment 6 also has two consecutive U-turn loops 7 creating enhanced flexibility of the frame structure 1. The two housings 9, 9' accommodate different parts of a device, e.g. a sensor device including a sensor unit, a battery for operating the sensor unit, an induction coil for charging the battery, and a processing unit. The electronic parts of the two housing may be connected via an electrical wire. By distributing the parts over two housings, the size of each individual housing may be reduced. In addition, it is possible to introduce more flexibility, e.g. by the U-turn loops 7, in the frame structure as would be the case, when one larger housing covers one large part of the central section 20 of the frame structure.

Fig. 6 shows the variant of the frame structure 1 of Fig. 3 arranged within an oral cavity of a user. The two larger black areas illustrate a device with two housings attached to the frame structure in its central section. The first distal section 31 is accommodated in an upper anterior vestibule of the user. The second distal section 41 is accommodated in a lower anterior vestibule of the user. The third and fourth distal sections 51, 51' are accommodated in a posterior vestibule of the user. The variants of Figs. 1, 3 and 4 are placed the same way within the oral cavity. Fig. 6 also shows pivot points of the jaw and the supporting oral cavity frame influencing the kinematic behaviour the supporting oral cavity frame.

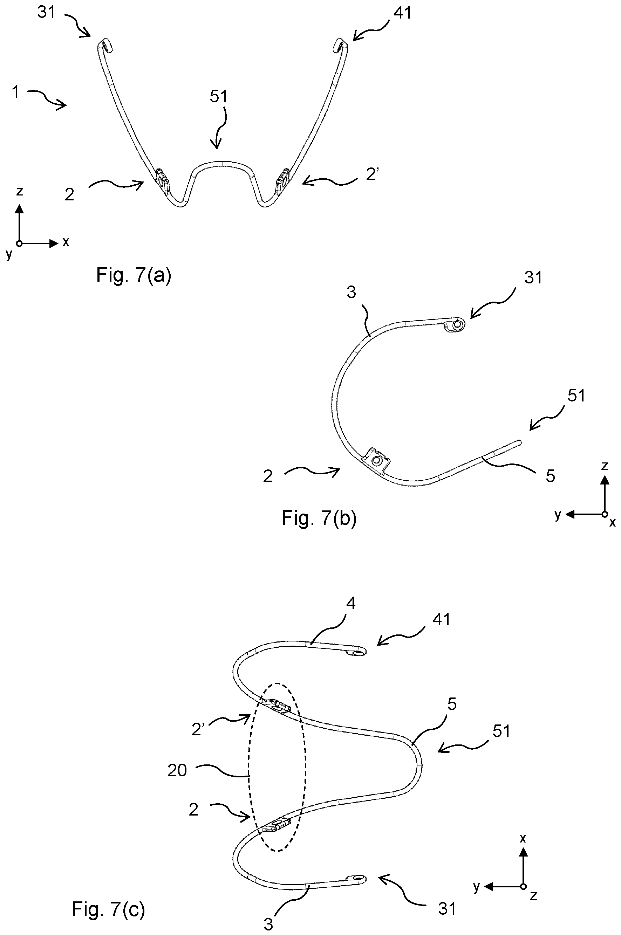

Fig. 7 shows a variant of a supporting oral cavity frame for sublingual placement with the same views as for Fig. 1. The supporting oral cavity frame of Fig. 7 differs from the supporting oral cavity frame of Fig. 2 in that it is for partially sublingual placement and not for placement in the left or right vestibule.

The wire-like frame structure 1 also has a central section from which three wire-like segments extend. In the installed state, a first distal section 31 of a first extending segment 3 is accommodated in an upper left anterior vestibule of the user, a second distal section 41 of a second extending segment 4 is accommodated in an upper right anterior vestibule of the user, and a third distal section 51 of a third extending segment 5 is accommodated sublingual. The first and second distal sections 31, 41 are formed by free ends. The third distal section 51 is formed by a loop. The third extending segment connects a first and second attachment area 2, 2' arranged within the central section 20 of the frame structure 1.

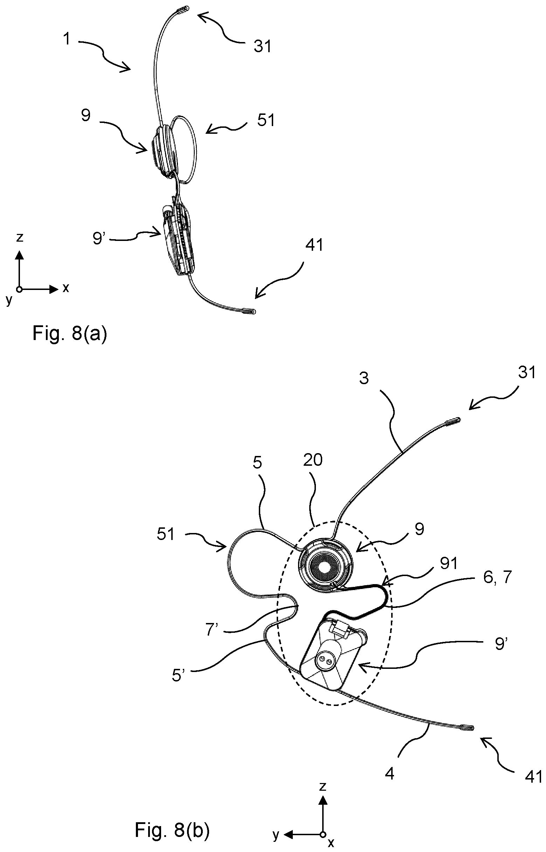

Fig. 8 shows an apparatus comprising the supporting oral cavity frame of the variant of Fig. 4 equipped with a sensor device accommodated in two housings 9, 9'. A first housing 9 is attached to the first attachment area 2 and comprises a battery and an inductive coil for charging the battery. A second housing 9' is attached to the second attachment area 2' and comprises a processing unit and the sensor. The two housings 9, 9' are electrically connected via a wire or flexible printed circuit board (PCB) 91, which is attached to the intermediate wire-like segment 6 between the two housings 9, 9'. The first housing 9 is supported in a circular opening formed by the frame structure 1 (see Fig. 4). The second housing 9' is glued or welded to the frame structure 1.

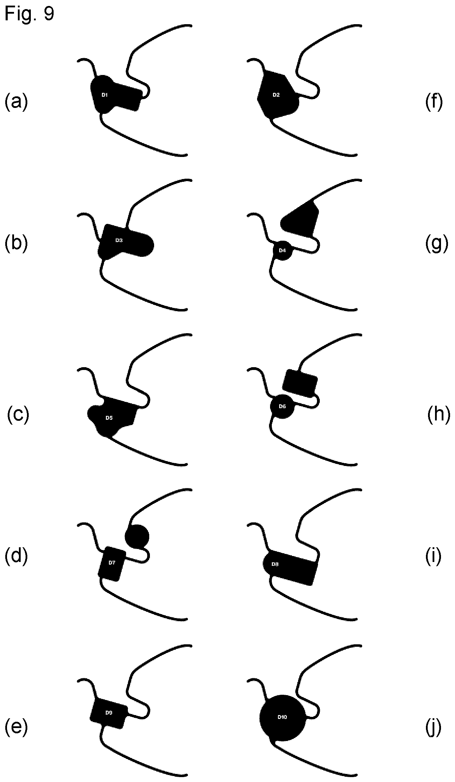

Fig. 9 shows under (a) to (j) several options for placing one (a, b, c, e, f, i, j) or two (d, g, h) housings (large black area) of a device on a frame structure of the variant of Fig. 1. The housings may have different shapes for accommodating the different parts of the device.

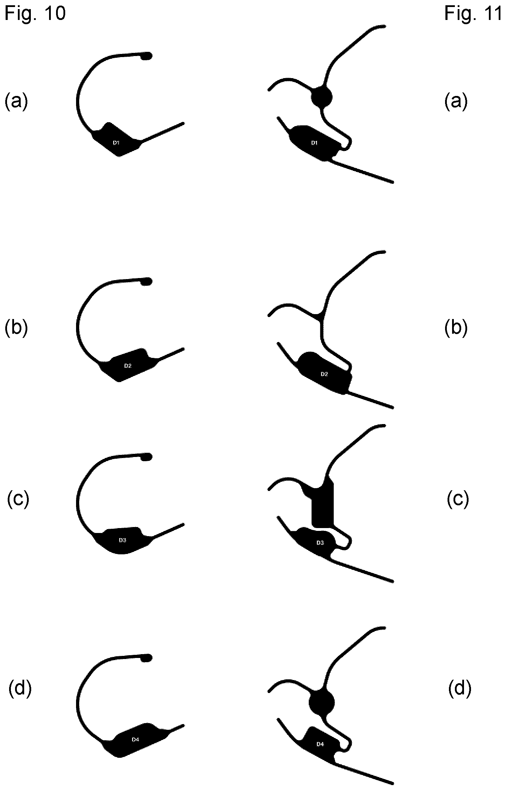

Fig. 10 shows under (a) to (d) several shapes of a housing (large black area) of a device on a frame structure of the variant of Fig. 7.

Fig. 11 shows under (a) to (d) several options for placing one (b) or two (a, c, d) housings (large black area) of a device on a frame structure of the variant of Fig. 3. The housings may have different shapes for accommodating the different parts of the device.

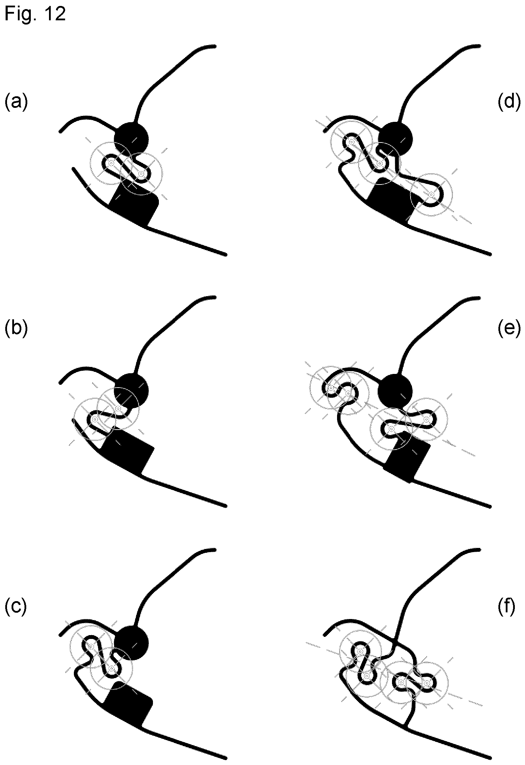

Fig. 12 shows under (a) to (f) frame structures with different U-turn loops to increase and adjust flexibility of the frame structure.

### Reference Signs

- 1: wire-like frame structure
- 2, 2': attachment area
- 20: central section
- 3: first extending wire-like segment
- 31: first distal section
- 4: second extending wire-like segment
- 41: second distal section
- 5, 5': third extending wire-like segment
- 51, 51': third distal section
- 6: intermediate wire-like segment
- 7: U-turn, loop
- 9, 9': device, housing
- 91: electrical connection, wire, flexible PCB

## Claims

1. Supporting oral cavity frame for carrying a device (9, 9') and for placement within an oral cavity of a user comprising
a wire-like frame structure (1) of resiliently flexible material;
wherein the frame structure (1) comprises a central section (20) with at least one attachment area (2, 2') for permanently or releasably attaching the device (9, 9') to the supporting frame (1)
and several wire-like segments (3, 4, 5, 5', 6);
wherein the several wire-like segments (3, 4, 5, 5') extend from the central section (20) and each of the several extending wire-like segments (3, 4, 5, 5') forms a distal section (31, 41, 51, 51');
wherein the wire-like frame structure (1) with the several extending segments (3, 4, 5, 5') is dimensioned and bent such that, in an installed state of the supporting frame (1) within the oral cavity, the distal sections (31, 41, 51, 51') of the several extending segments (3, 4, 5, 5') rest against different areas of tissue within the oral cavity to stabilize the supporting oral cavity frame with the at least one attachment area (2, 2') at a desired location within the oral cavity.

2. Supporting oral cavity frame according to claim 1, wherein at least three wire-like segments (3, 4, 5, 5') extend from the central section (20) and each of the at least three extending wire-like segments (3, 4, 5, 5') forms a distal section (31, 41, 51, 51'); wherein the wire-like frame structure (1) with the at least three extending segments (3, 4, 5, 5') is dimensioned and bent such that, in an installed state of the supporting frame (1) within the oral cavity, the distal sections (31, 41, 51, 51') of the at least three extending segments (3, 4, 5, 5') rest against three different areas of tissue within the oral cavity to stabilize the at least one attachment area (2, 2') at a desired location within the oral cavity.

3. Supporting oral cavity frame according to one of the preceding claims, wherein the distal sections (31, 41, 51, 51') of the wire-like extending segments (3, 4, 5, 5') are formed by free ends (31, 41) or loops (51) and preferably extend in different directions from the central section (20).

4. Supporting oral cavity frame according to one of the preceding claims, wherein the wire-like frame structure (1) comprises an intermediate wire-like segment (6) connecting two attachment areas (2, 2') and/or extending wire-like segments (3, 4, 5, 5').

5. Supporting oral cavity frame according to one of the preceding claims, wherein the at least one attachment area (2, 2') is formed by or at connection points or junctions of the wire-like segments (3, 4, 5, 5', 6) or by bends or loops in the wire-like segment (3, 4, 5, 5', 6) within the central section (20).

6. Supporting oral cavity frame according to one of the preceding claims, wherein the wire-like segments (5, 6) comprise U-turn loops (7) to form pivot points increasing resilient flexibility within the frame structure.

7. Supporting oral cavity frame according to one of the preceding claims, wherein the frame structure (1) is made from one bent wire or several bent wires connected to each other, preferably a spring steel wire.

8. Supporting oral cavity frame according to one of the preceding claims, wherein the frame structure (1) is coated with a biocompatible material, preferably a soft and elastic material.

9. Supporting oral cavity frame according to one of the preceding claims, wherein the wire-like frame structure (1) with the at least three extending wire-like segments (3, 4, 5, 5') is dimensioned and bent such that, a first distal section (31) of a first extending segment (3) is accommodated in an upper anterior vestibule of the user, a second distal section (41) of a second extending segment (4) is accommodated in a lower anterior vestibule of the user, and a third distal section (51) of a third extending segment (5) is accommodated in a posterior vestibule of the user.

10. Supporting oral cavity frame according to claim 9, wherein the central section (20) lies approximately in a plane and the extending segments (3, 4, 5, 5') with the distal sections (31, 41, 51, 51') are bent in the same direction out of the plane.

11. Supporting oral cavity frame according to one of claims 1 to 8, wherein the wire-like frame structure with the at least three extending wire-like segments is dimensioned and bent such that, in the installed state of the supporting frame within the oral cavity, a first distal section (31) of a first extending segment (3) is accommodated in an upper left anterior vestibule of the user, a second distal section (41) of a second extending segment (4) is accommodated in an upper right anterior vestibule of the user, and a third distal section (51) of a third extending segment (5) is accommodated sublingually.

12. Apparatus comprising the supporting oral cavity frame of one of the preceding claims and a device (9, 9') attached to the at least one attachment area (2, 2') within the central section (20) of the frame structure (1), wherein the device (9, 9') is a sensor or monitoring device or a drug delivery device.

13. Apparatus according to claim 12, wherein the device (9, 9') comprises at least one housing, a sensor unit or a drug-delivery unit, a battery for operating the sensor unit, an induction coil for charging the battery, and a processing unit.

14. Apparatus according to claim 12 or 13, wherein the device comprises two separate housings (9, 9') accommodating different parts of the device and connected via an electrical connection (91).

15. Method of placing a device (9, 9') within an oral cavity of a user comprising the steps of:
a. providing a supporting oral cavity frame of one of the preceding claims;
b. attaching the device (9, 9') to the frame structure (1) to obtain an apparatus according to one of the claims 12 to 14;
c. placing the supporting oral cavity frame within the oral cavity such that distal sections (31, 41, 51) rest against different areas of tissue within the oral cavity to stabilize the device at a desired location within the oral cavity of the user.
